# EUROPÄISCHE PATENTSCHRIFT

(11) **EP 0 915 728 B1**
(45) Veröffentlichungstag und Bekanntmachung des Hinweises auf die Patenterteilung: **20.11.2002**
(21) Anmeldenummer: 97918957.8
(22) Anmeldetag: 04.08.1997
(51) Int. Cl.: B01D 17/02, C07C 57/04

(54) **VORRICHTUNG ZUR PHASENTRENNUNG**
DEVICE FOR PHASE SEPARATION
DISPOSITIF POUR LA SEPARATION DE PHASES

(30) Priorität: 05.08.1996 DE 19631662
(43) Veröffentlichungstag der Anmeldung: 19.05.1999
(73) Patentinhaber: BASF AKTIENGESELLSCHAFT, 67056 Ludwigshafen (DE)
(72) Erfinder: HEIDA, Bernd, D-67158 Ellerstadt (DE); THIESSEN, Fritz, D-67071 Ludwigshafen (DE); HAMMON, Ulrich, D-68163 Mannheim (DE)
(74) Vertreter: Isenbruck, Günter
(86) Internationale Anmeldenummer: EP9704239
(87) Internationale Veröffentlichungsnummer: WO98005398

(56) Entgegenhaltungen:
- EP-A- 0 706 986
- EP-A- 0 778 255
- DE-U- 1 915 931
- SOVIET INVENTIONS ILLUSTRATED Week 8524 25.Juli 1985 Derwent Publications Ltd., London, GB; AN 85145091 XP002050870 & SU 1 126 309 A (PETROL GAS IND RES) , 30.November 1984

## Beschreibung

Die vorliegende Erfindung bezieht sich auf einen Abscheider mit verbesserter Trennung von Gemischen aus Flüssigkeiten unterschiedlicher Dichte. Die Erfindung betrifft darüber hinaus ein durch den Einsatz des erfindungsgemäßen Abscheiders verbessertes Verfahren zur Acrylsäureherstellung durch katalytische Gasphasenoxidation.

In vielen Bereichen der chemischen Industrie werden Abscheider zur Trennung zweier nicht beliebig ineinander lösbarer Flüssigkeiten unterschiedlicher Dichte verwendet. Solche Abscheider sind liegende oder stehende Behälter mit einem Zulauf für das zu trennende Gemisch auf der einen Seite und einem höher und einem tiefer angebrachten Ablauf für die getrennten unterschiedlich dichten Phasen auf der gegenüberliegenden Seite. Bei bekannten Abscheidern ist der Zulauf des Gemisches, also das Einleitungsrohr in den Behälter, gerade ausgestaltet und meistens horizontal angeordnet. Das dem Abscheider zugeleitete Flüssigkeitsgemisch strömt somit mit einem erheblichen horizontalen Impuls in den Abscheidebehälter ein. Dadurch wird die Phasentrennung, insbesondere die Ausbildung einer Phasengrenze, erschwert. Um diesen Nachteil zu vermeiden, wurden Abscheider mit einem großen Verhältnis von Länge zu Durchmesser vorgeschlagen, so daß der horizontale Einleitungsimpuls in der Flüssigkeit abgebaut wird. Nachteil dieser Lösung ist, daß ein nicht unerheblicher Teil der Baulänge des Abscheiders nicht für die Phasentrennung, sondern für die Abbremsung der zugeleiteten Flüssigkeit verwendet wird. Eine andere Lösung besteht darin, in dem Abscheidebehälter in der Nähe des Zulaufs ein oder mehrere Prallbleche anzubringen, um den Impuls der eingeleiteten Flüssigkeit abzubauen, was bereits aus anderen Strömungsanlagen bekannt ist. Nachteil dieser Lösung ist der erheblich höhere Bauaufwand für den Abscheider, denn die Prallbleche müssen zur Erzielung einer positiven Wirkung strömungstechnisch genau überlegt und exakt eingebaüt sein. Trotzdem können unerwünschte Turbulenzen meist nicht vollständig verhindert werden.

Aus US-A-4,844,801 ist ein Abscheider bekannt wobei das aufzutrennende Gemisch auf die den Abläufen gegenüberliegende Wand senkrecht auftrifft. Der Abscheider weist im Bereich der Einleitung des aufzutrennenden Flüssigkeitsgemisches jalousienartige, schräggestellte Leitlamellen auf, die eine richtungsgebende Funktion für das eingeleitete Gemisch haben. Sie können jedoch keinen Druckverlust in der Flüssigkeit aufbauen und auch keine Wirbel abbauen, somit auch nicht die Strömung vergleichmäßigen. Der eingeleitete Impuls wird zwar gezielt umgelenkt, er wird aber nicht abgebaut.

Ein besonders interessanter Anwendungsbereich für einen Abscheider besteht bei der Abtrennung des Lösungsmittels der Acrylsäure-Absorption aus Sauerwasser, das bei der katalytischen Gasphasenoxidation von Propen entsteht. Bei diesem Verfahren zur Herstellung von Acrylsäure wird das zu oxidierende Propen mit einem Verdünnungsgas oder mit einem nicht umgesetzte Edukte enthaltenden Kreisgas vermischt und dann der Gasphasenoxidation zugeführt. Das Reaktionsgemisch der Gasphasenoxidation wird zur Abtrennung der Acrylsäure in eine Absorptionskolonne geleitet. Dort wird der überwiegende Teil der entstandenen Acrylsäure mit Hilfe eines Lösungsmittels aus dem Reaktionsgemisch abgetrennt. Die nicht absorbierten Komponenten werden gasförmig aus der Absorptionskolonne entnommen und einer Kondensationsstufe zugeführt. Der kondensierbare Teil dieses Gasgemischs wird in kondensierter Form abgetrennt und als sogenanntes Sauerwasser abgeleitet. Der nicht kondensierbare gasförmige Teil wird teilweise wieder in die Gasphasenoxidation zurückgeführt und bildet das sogenannte Kreisgas. Das Sauerwasser wird im Regelfall verbrannt. Mit dem Sauerwasser werden auch erhebliche Restmengen an Lösungsmittel entfernt, das dann im Lösungsmittelkreislauf wieder ergänzt werden muß.

Aufgabe der vorliegenden Erfindung ist es deshalb, einen Abscheider bereitzustellen, bei dem mit einfachen konstruktiven Mitteln eine bessere Phasentrennung erreicht wird. Eine weitere Aufgabe besteht darin, ein verbessertes Verfahren zur Herstellung von Acrylsäure durch katalytische Gasphasenoxidation bereitzustellen, wobei die Verluste an im Sauerwasser enthaltenem Lösungsmittel deutlich reduziert sind.

Die Lösung geht aus von einem Abscheider zur Trennung eines Gemisches aus Flüssigkeiten verschiedener Dichte, der einen Behälter mit mindestens einem Zulauf für die Zuleitung des Gemisches, einen höher liegenden Ablauf für die Ableitung der leichteren Phase des Gemisches und einen niedriger liegenden Ablauf für die Ableitung der schwereren Phase des Gemisches enthält. Der Zulauf ist derart angeordnet und ausgebildet, daß das eingeleitete Gemisch auf eine den Abläufen abgewandte Innenwand des Behälters in einem Winkel α von 0° bis 45°, vorzugsweise von 0° bis 20°, besonders bevorzugt von 0° bis 5° zu einer Achse, die senkrecht auf der Innenwand steht, auf die Innenwand auftrifft. Der erfindungsgemäße Abscheider ist dann dadurch gekennzeichnet, daß in dem Behälter Zwischen dem Zulauf und den Abläufen ein Lochblech angebracht ist und daß das Loch blech den Behälter senkrecht zur Achse in seinem gesamten Querschnitt teilt.

Dabei sind der Zulauf und die Abläufe in dem Behälter an voneinander entfernten, im Regelfall an gegenüberliegenden Seiten, angebracht. Die genannte Achse steht auf der betreffenden Behälterwand bzw., bei gekrümmter Behälterwand, auf der Auftrefffläche des Flüssigkeitsstrahls senkrecht. Vorzugsweise trifft der Flüssigkeitsstrahl senkrecht, also mit einem Winkel von 0° auf die Behälterwand. Eine schräge Anströmung verschlechtert den Effekt des Impulsabbaus an der Behälterwand, da die quer zur Wandfläche gerichtete Impulskomponente nicht durch die Wand, sondern nur durch die umgebende Flüssigkeit abgebaut werden kann, was Abbremsraum benötigt und zu unerwünschten Wirbeln führen kann. Die beschriebene Umlenkung des zugeführten Flüssigkeitsstroms auf eine Wand des Trennbehälters führt nahezu zu einem völligen Abbau der zur Wand senkrechten Impulskomponente, so daß die von der Innenwand zurückströmende Flüssigkeit nur noch den zur Einströmung in den eigentlichen Entmischungsbereich des Abscheiders notwendigen Restimpuls senkrecht zur Wand besitzt. Die gute Wirkung dieser Konstruktion ist überraschend, denn bei der weitgehend elastischen Reflexion der Flüssigkeitsteilchen an der harten Behälterwand erwartet man eine Impulsänderung bei Energieerhaltung, also im wesentlichen eine Umkehrung der Bewegungsrichtung der Flüssigkeitsteilchen unter Beibehaltung des Impulsbetrages. Wie beim Aufprall eines Wasserstrahls an eine Wand wäre also eher ein Zurückprallen der Flüssigkeit zu erwarten. Die Erklärung für den unerwarteten Effekt liegt vermutlich darin, daß die gesamte Energie des einlaufenden Flüssigkeitsstrahls in einem stationären kreisförmigen Wirbel zwischen der Behälterwand und dem Einlauf abgebaut wird, also keine freien Wirbel bestehen.

Vorzugsweise wird in dem Behälter zwischen dem Zulauf und den beiden Abläufen ein Lochblech angebracht. Das Lochblech vergleichmäßigt den Flüssigkeitsstrom in dem Behälter, insbesondere baut es eventuell infolge der zentralen Einleitung bestehende radiale Druckdifferenzen ab, die ansonsten zur Bildung großräumiger Wirbel und damit zu verringerter Trennleistung führen könnten. Dieses Lochblech teilt den Behälter vorzugsweise parallel zu der Behälterwand, auf die der einlaufende Strahl trifft, in seinem gesamten Querschnitt, es erstreckt sich also durch den ganzen Behälter. Es kann aber auch ein Lochblech verwendet werden, das etwa einen mehr oder weniger breiten Randspalt an der Behälterwand frei läßt. Das Lochblech wird im übrigen am besten nahe an der Zuleitung angebracht, so daß ein möglichst großer wirbelfreier Trennbereich in dem Behälter entsteht. Vorzugsweise weist das Lochblech ein Flächenverhältnis der Durchlaßöffnungen zur Gesamtfläche von 1% bis 40%, vorzugsweise von 3% bis 15% auf.

Entsprechend den obigen Ausführungen wird auch ein Verfahren zur Trennung von Gemischen aus nicht beliebig ineinander löslichen Flüssigkeiten unterschiedlicher Dichte bereitgestellt. Dieses Verfahren kann bevorzugt für die Trennung sehr unterschiedlicher Flüssigkeiten, zum Beispiel von wäßrigen und organischen Flüssigkeiten in einem entsprechenden Gemisch eingesetzt werden.

Erfindungsgemäß kann der beschriebene Abscheider in einem Verfahren zur Herstellung von Acrylsäure durch katalytische Gasphasenoxidation von Propen verwendet werden, wobei aus dem Reaktionsgemisch der Gasphasenoxidation Acrylsäure in einer Absorptionsstufe mit Hilfe eines ersten Lösungsmittels absorbiert wird, anschließend aus der Absorptionsstufe ein Gasgemisch entnommen wird, das an dem ersten Lösungsmittel und an Acrylsäure arm ist, dann in einer Kondensationsstufe das Gasgemisch auf eine Temperatur zwischen 20 °C und 60 °C abgekühlt wird, die kondensierte Phase des Gasgemisches aus der Kondensationsstufe als Sauerwasser entnommen wird und schließlich die gasförmige Phase des Gasgemisches aus der Kondensationsstufe entnommen und zumindest teilweise als Kreisgas zur Gasphasenoxidation zurückgeführt wird. Erfindungsgemäß werden dabei Mittelsieder, insbesondere Maleinsäureanhydrid, mit Hilfe des Sauerwassers aus dem ersten Lösungsmittel dadurch abgetrennt, daß Sauerwasser und mindestens ein ein Teilstrom des ersten Lösungsmittels einer Mischvorrichtung und anschließend einem erfindungsgemäßen Abscheider zugeführt werden. Als erstes Lösungsmittel wird dabei vorzugsweise ein Lösungsmittel verwendet, das mindestens ein substituiertes oder unsubstituiertes Diphenyl, einen substituierten oder unsubstituierten Diphenylether oder Dimethylphthalat enthält. Weiter bevorzugt ist eine Mischung aus etwa 60 Gew.-% Diphenylether mit etwa 20 Gew.-% Diphenyl und 20 Gew.-% Dimethylphthalat.

Die vorliegende Erfindung wird im folgenden anhand der Figuren 1 bis 3 beschrieben. Dabei zeigen:
- Figur 1:: Beispiel für einen Abscheider nach dem Stand der Technik;
- Figur 2:: Beispiele für einen erfindungsgemäßen Abscheider;
- Figur 3:: Beispiel eines erfindungsgemäßen Verfahrens zur Herstellung von Acrylsäure.

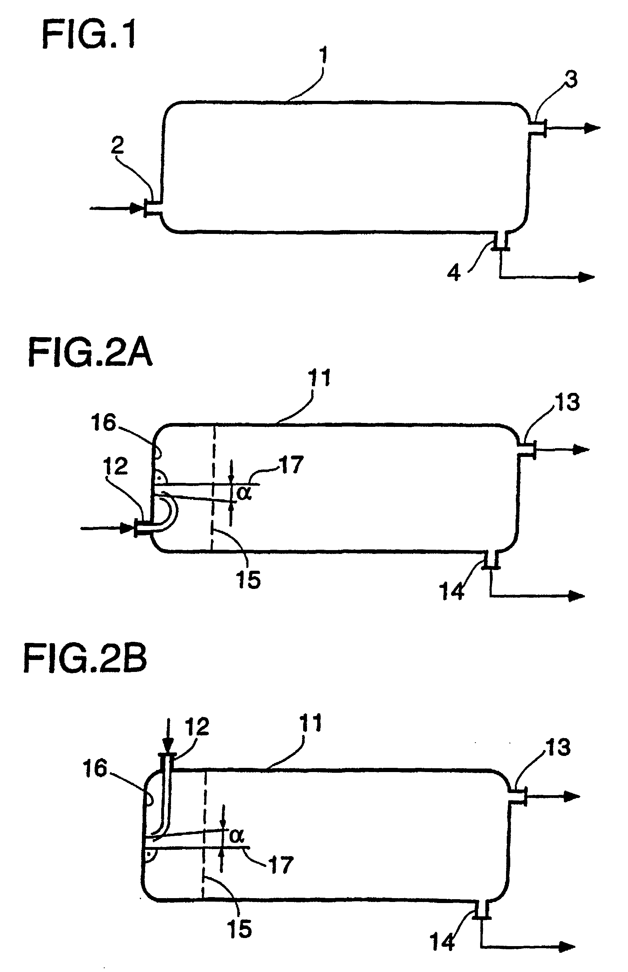

Figur 1 zeigt einen bereits bekannten Abscheider. Ein Behälter 1 weist einen Zulauf 2 für die zu trennende Flüssigkeit und zwei Abläufe 3 und 4 für die getrennten Phasen auf. Zulauf 2 ist einfach plan an eine Behälterwand angesetzt. Bei anderen Ausführungsformen kann der Zulauf 2 auch in den Behälter hineinragen oder schräg angeordnet sein oder es können Prallbleche in dem Behälter 1 derart angebracht sein, daß der Impuls des eingeleiteten Flüssigkeitsgemischs weitgehend abgebaut wird.

Figur 2A zeigt dagegen einen erfindungsgemäßen Abscheider. Der Zulauf 12 in den Behälter 11 ist hier nach rückwärts gebogen und derart angeordnet, daß der eingeleitete Flüssigkeitsstrahl unter dem Winkel α zur Achse 17 auf die Behälterinnenwand 16 trifft, die den Abläufen 13 und 14 gegenüberliegt. Dabei schließt die Achse 17 im Auftreffbereich des einlaufenden Flüssigkeitsstrahls einen Winkel von 90° mit der Behälterwand 16 ein. Zusätzlich ist in dem Behälter 11 ein Lochblech 15 angebracht, wobei die Lochfläche etwa 10% der Gesamtfläche des Lochblechs ausmacht. Durch den gebogenen Einlauf 12 wird der horizontale Eingangsimpuls fast vollständig abgebaut. Das Lochblech 15 vergleichmäßigt dann den Flüssigkeitsstrom weiter und definiert mit den Innenwänden des Behälters 11 einen Trennraum, in dem eine sehr viel effizientere Phasentrennung stattfindet als in herkömmlichen Abscheidern. Statt einem Einlauf 12 können natürlich auch mehrere Einläufe verwendet werden, die auf die gleiche oder auf verschiedene Innenwände des Behälters gerichtet sind. Figur 2B zeigt eine Variante des in Figur 2A dargestellten und eben beschriebenen Abscheiders. Dabei wird das Flüssigkeitsgemisch über Leitung 12 von oben eingeleitet. Genausogut könnte es auch von unten in den Behälter 11 eingebracht werden, sofern nur die obigen Bedingungen für das Auftreffen auf der Behälterwand 16 erfüllt sind.

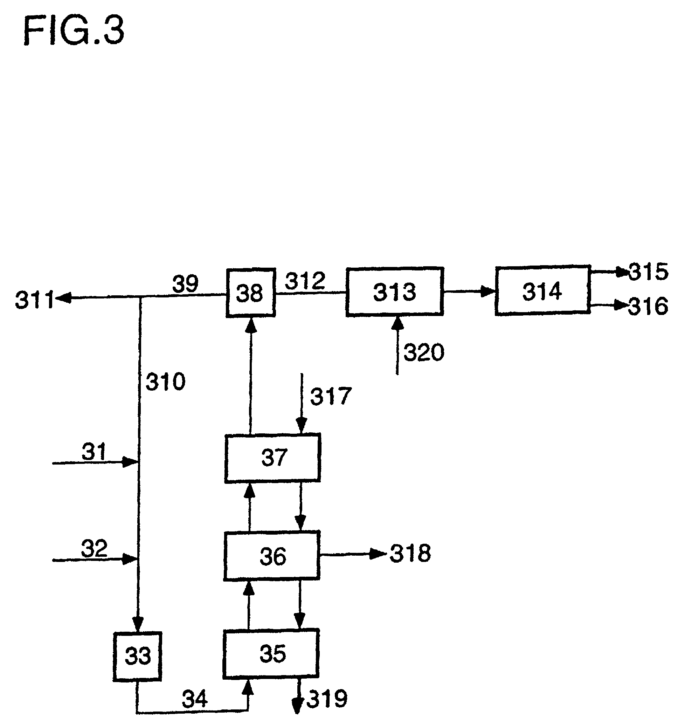

Figur 3 stellt eine Prinzipskizze eines erfindungsgemäß durch den Einsatz eines oben beschriebenen Abscheiders verbesserten Verfahrens zur Herstellung von Acrylsäure durch katalytische Gasphasenoxidation dar. Dabei wird Propen über eine Leitung 31 und ein Verdünnungsgas, zum Beispiel Luft oder Wasserdampf über eine Leitung 32 einem Reaktor 33 zugeführt, in dem die katalytische Gasphasenoxidation von Propen abläuft. Das dabei entstehende Acrolein kann in einem weiteren, nicht dargestellten Reaktor oxidiert werden. Über eine Leitung 34 gelangt das Reaktionsgemisch der Gasphasenoxidation in einen Quenchapparat 35. Dort wird das Reaktionsgemisch abgekühlt und ein Teil des Absorptionsmittels (Lösungsmittel) verdampft, das über eine Leitung 317 einer Absorptionskolonne 37 zugeführt wird und von dort über eine Kühleinheit 36 zum Quenchapparat 35 geleitet wird. Schwersiedende Nebenkomponenten des Lösungsmittels werden im Quenchapparat 35 kondensiert und über eine Entnahmeleitung 319 abgezogen. Sie werden, gegebenenfalls nach einer Abdestillation von Lösungsmittel, entsorgt, zum Beispiel verbrannt. Das bereits stark abgekühlte Reaktionsgemisch wird vom Quenchapparat 35 weiter zur Kühleinheit 36, die zum Beispiel aus Kühlkreisläufen besteht, geleitet, wo es auf die geeignete Absorptionstemperatur gekühlt wird. Anschließend wird das Reaktionsgemisch in die Absorptionskolonne 37 geleitet. Dort wird durch Gegenstromabsorption mit dem über Leitung 317 zugeführten Lösungsmittel Acrylsäure aus dem Reaktionsprodukt der Gasphasenoxidation abgetrennt. Das Lösungsmittel kann zum Beispiel aus einem Gemisch aus 75 Gew.-% Diphenylether und 25 Gew.-% Diphenyl bestehen. Es kann darüber hinaus auch bis zu 25 Gew.-% polare Lösungsmittel zur Reduktion des Feststoffanfalls aufweisen, zum Beispiel Dimethylphthalat. Das mit Acrylsäure beladene Lösungsmittel wird in Kühleinheit 36 geleitet und aus dieser über einen Seitenabzug 318 zur weiteren, hier nicht dargestellten, Aufbereitung abgezogen. Diese Aufbereitung enthält regelmäßig eine Leichtsiederstrippung und notwendigerweise eine Lösungsmitteldestillation oder einen vergleichbaren Verfahrensschritt. Die von Acrylsäure weitgehend befreiten Reaktionsprodukte werden aus der Absorptionskolonne 37 über Kopf abgezogen und in einen Quenchapparat 38 geleitet. Dort wird der nicht-kondensierbare Teil dieser Reaktionsprodukte über eine Leitung 39 entnommen und, nach der Abtrennung und Ableitung von Inertgaskomponenten über eine Leitung 311, als Kreisgas über eine Leitung 310 zur Gasphasenoxidation von Propen zurückgeführt. Dieses Kreisgas enthält unter anderem nicht umgesetzte Edukte der Gasphasenoxidation, Stickstoff und Kohlenstoffoxide. Der kondensierbare Teil der von Acrylsäure befreiten Reaktionsprodukte wird über eine Leitung 312 entnommen. Dieses als Sauerwasser bezeichnete Kondensat besteht aus einer wäßrigen Lösung, die neben Acrylsäure noch relevante Mengen an Essigsäure, Maleinsäure und Formaldehyd, sowie weitere Säuren enthält. Das Sauerwasser wird dann über Leitung 312 einer Mischeinheit 313 zugeführt. Dort wird das Sauerwasser mit einem Teilstrom des ersten Lösungsmittels, der über Leitung 320 herangeführt wird und von der Aufbereitung des mit Acrylsäure beladenen Lösungsmittelstroms kommt, vermischt. Anschließend wird das Gemisch einem erfindungsgemäßen Abscheider 314 zugeleitet. Aus dem Abscheider werden dann die getrennten Phasen über Leitungen 315 und 316 entnommen. Bei diesem Verfahren zur Herstellung von Acrylsäure konnte der Verlust an Lösungsmittel durch die Verwendung des erfindungsgemäßen Abscheiders etwa auf ein Drittel gesenkt werden.

Diese Hauptschritte der Acrylsäureherstellung können durch eine Vielzahl weiterer Schritte ergänzt werden, die jedoch die Bedeutung der vorliegenden Erfindung nicht mindern. Das erfindungsgemäß ausgestaltete Verfahren stellt somit einen effizienten Weg zur Herstellung von Acrylsäure dar, wobei die Lösungsmittelverluste deutlich, nämlich um bis zu 70%, reduziert sind. Im übrigen wird im Rahmen der Erfindung allgemein eine gleichermaßen einfache und wirksame Vorrichtung zur Phasentrennung bereitgestellt.

## Patentansprüche

1. Abscheider zur Trennung eines Gemisches aus Flüssigkeiten unterschiedlicher Dichte, der einen Behälter 11 mit mindestens einem Zulauf 12 für die Zuleitung des Gemisches, einen höher liegenden Ablauf 13 für die Ableitung der leichteren Phase des Gemisches und einen niedriger liegenden Ablauf 14 für die Ableitung der schwereren Phase des Gemisches enthält, wobei der Zulauf 12 derart angeordnet und ausgebildet ist, daß das eingeleitete Gemisch auf eine den Abläufen 13 und 14 abgewandte Innenwand 16 des Behälters 11 in einem Winkel α von 0° bis 45° zu einer Achse 17, die senkrecht auf der Innenwand 16 steht, auf die Innenwand 16 auftrifft, **dadurch gekennzeichnet, daß** in dem Behälter 11 zwischen dem Zulauf 12 und den Abläufen 13 und 14 ein Lochblech 15 angebracht ist und daß das Lochblech 15 den Behälter 11 senkrecht zur Achse 17 in seinem gesamten Querschnitt teilt.

2. Abscheider nach Anspruch 1, **dadurch gekennzeichnet, daß** das Lochblech 15 im Behälter 11 einen Randspalt an der Behälterwand freiläßt.

3. Abscheider nach Anspruch 1 oder 2, **dadurch gekennzeichnet, daß** das Lochblech 15 ein Flächenverhältnis der Durchlaßöffnungen zur Gesamtfläche des Lochblechs von 1% bis 40%, vorzugsweise von 3% bis 15% aufweist.

4. Verwendung eines Abscheiders nach einem der Ansprüche 1 bis 3 in einem Verfahren zur Herstellung von Acrylsäure durch katalytische Gasphasenoxidation von Propen, wobei
aus dem Reaktionsgemisch der Gasphasenoxidation Acrylsäure in einer Absorptionsstufe mit Hilfe eines ersten Lösungsmittels absorbiert wird, aus der Absorptionsstufe ein Gasgemisch entnommen wird, das an dem ersten Lösungsmittel und an Acrylsäure arm ist,
in einer Kondensationsstufe das Gasgemisch, vorzugsweise auf eine Temperatur zwischen 20°C und 60°C, abgekühlt wird,
die kondensierte Phase des Gasgemisches aus der Kondensationsstufe als Sauerwasser entnommen wird,
die gasförmige Phase des Gasgemisches aus der Kondensationsstufe entnommen und zumindest teilweise als Kreisgas zur Gasphasenoxidation zurückgeführt wird, und
das Sauerwasser zusammen mit mindestens einem Teilstrom des ersten Lösungsmittels einer Mischvorrichtung und anschließend dem Abscheider zugeleitet wird, um Mittelsieder, insbesondere Maleinsäureanhydrid, aus dem ersten Lösungsmittel abzutrennen.

5. Verwendung nach Anspruch 4, **dadurch gekennzeichnet, daß** das erste Lösungsmittel mindestens ein substituiertes oder unsubstituiertes Diphenyl, einen substituierten oder unsubstituierten Diphenylether oder Dimethylphthalat enthält.

6. Verfahren zur Trennung von Gemischen von Flüssigkeiten mit unterschiedlicher Dichte, wobei das Gemisch über einen Zulauf 12 in einen Behälter 11 geleitet wird und während einer zur Trennung der Phasen des Gemisches notwendigen Zeit darin verweilt und wobei anschließend die schwerere Phase des getrennten Gemisches über einen Ablauf 14 und die leichtere Phase des getrennten Gemisches über einen Ablauf 13 aus dem Behälter entnommen wird, wobei der Zulauf 12 in dem Behälter 11 derart angeordnet und ausgebildet ist, daß das eingeleitete Gemisch auf eine den Abläufen 13 und 14 abgewandte Innenwand 16 des Behälters 11 in einem Winkel α von 0° bis 45° zu einer Achse 17 die senkrecht auf der Innenwand 16 steht, auf die Innenwand 16 auftrifft, **dadurch gekennzeichnet, dass** im Behälter 11 zwischen dem Zulauf 12 und den Abläufen 13 und 14 ein Lochblech 15 angeordnet ist und dass das Lochblech 15 den Behälter 11 senkrecht zur Achse 17 in seinem gesamten Querschnitt teilt.

7. Verfahren nach Anspruch 6, **dadurch gekennzeichnet, daß** das Gemisch von Flüssigkeiten aus mindestens einer organischen Phase und einer wäßrigen Phase besteht.

## Claims

1. A separator for separating a mixture of liquids of different densities, which separator comprises a vessel 11 having at least one inlet 12 for feeding the mixture, a higher outlet 13 for discharging the lighter phase of the mixture and a lower outlet 14 for discharging the denser phase of the mixture, in which the inlet 12 is arranged and constructed in such a manner that the introduced mixture impacts an inner wall 16 of the vessel 11 opposite the outlets 13 and 14 at an angle α of from 0° to 45°, to an axis 17 which is perpendicular to the inner wall 16 wherein a perforated plate 15 is mounted in the vessel 11 between the inlet 12 and the outlets 13 and 14 and the perforated plate 15 divides the vessel 11 perpendicularly to the axis 17 over its entire cross section.

2. A separator as claimed in claim 1, wherein the perforated plate 15 leaves an edge gap open at the vessel wall in the vessel 11.

3. A separator as claimed in claim 1 or 2, wherein the perforated plate 15 has an area ratio of the through-holes to the total area of the perforated plate of from 1% to 40%, preferably of from 3% to 15%.

4. The use of a separator as claimed in one of claims 1 to 3, in a process for preparing acrylic acid by catalytic gas-phase oxidation of propene,
acrylic acid being absorbed from the gas-phase oxidation reaction mixture using a first solvent in an absorption stage, a gas mixture which is low in the first solvent and acrylic acid being taken off from the absorption stage,
the gas mixture being cooled, preferably to from 20°C to 60°C, in a condensation stage,
the condensed phase of the gas mixture being taken off as acid water from the condensation stage,
the gaseous phase of the gas mixture being taken off from the condensation stage and recycled at least in part to the gas-phase oxidation as circulating gas, and
the acid water, together with at least a part-stream of the first solvent, being fed to a mixing apparatus and then to the separator, in order to separate off medium-boilers, in particular maleic anhydride, from the first solvent.

5. The use as claimed in claim 4, wherein the first solvent comprises at least one substituted or unsubstituted biphenyl, a substituted or unsubstituted diphenyl ether or dimethyl phthalate.

6. A process for separating mixtures of liquids having different densities, the mixture being passed via an inlet 12 into a vessel 11 and residing therein during a time necessary for the separation of the phases of the mixture and the denser phase of the separated mixture then being taken off from the vessel via an outlet 14 and the lighter phase of the separated mixture then being taken off from the vessel via an outlet 13, which comprises the inlet 12 being arranged and constructed in the vessel 11 in such a manner that the introduced mixture impacts an inner wall 16 of the vessel 11 opposite the outlets 13 and 14 at an angle α of from 0° to 45° to an axis 17 which is perpendicular to the inner wall 16, wherein a perforated plate 15 is arranged in the vessel 11 between the inlet 12 and the outlets 13 and 14 and the perforated plate 15 divides the vessel 11 perpendicularly to the axis 17 over its entire cross section.

7. A process as claimed in claim 6, wherein the mixture of liquids comprises at least one organic phase and an aqueous phase.

## Revendications

1. Dispositif de séparation pour séparer un mélange de liquides de densités différentes, qui comprend un récipient (11) avec au moins une entrée (12) pour l'amenée du mélange, une sortie (13) située plus haut pour la dérivation de la phase légère du mélange et une sortie (14) située plus bas pour la dérivation de la phase lourde du mélange, l'entrée (12) étant agencée et réalisée de manière que le mélange introduit frappe une paroi interne (16) du récipient (11) qui est située à l'opposé des sorties (13) et (14), sous un angle α de 0° à 45° par rapport à un axe (17) qui est perpendiculaire à la paroi interne (16), **caractérisé en ce qu'**une plaque perforée (15) est agencée dans le récipient (11) entre l'entrée (12) et les sorties (13 et 14) et **en ce que** la plaque perforée (15) partage le récipient (11) perpendiculairement à l'axe (17) dans la totalité de sa section transversale.

2. Dispositif de séparation suivant la revendication 1, **caractérisé en ce que** la plaque perforée (15) du récipient (11) laisse libre une fente marginale à la paroi du récipient.

3. Dispositif de séparation suivant l'une des revendications 1 et 2, **caractérisé en ce que** la plaque perforée (15) présente un rapport entre la surface des ouvertures de passage et la surface totale de la plaque perforée de 1% à 40%, de préférence de 3% à 15%.

4. Utilisation d'un dispositif de séparation suivant l'une des revendications 1 à 3, dans un procédé de préparation d'acide acrylique par oxydation en phase gazeuse catalytique de propène, dans lequel
de l'acide acrylique est, dans une étape d'absorption, absorbé à partir du mélange réactionnel de l'oxydation en phase gazeuse à l'aide d'un premier solvant, un mélange gazeux, qui est pauvre en le premier solvant et en acide acrylique, étant prélevé de l'étape d'absorption,
le mélange gazeux est, dans une étape de condensation, refroidi de préférence à une température comprise entre 20°C et 60°C,
la phase condensée du mélange gazeux est prélevée de l'étape de condensation sous la forme d'eau acide,
la phase gazeuse du mélange gazeux est prélevée de l'étape de condensation et est recyclée au moins partiellement sous la forme de gaz de recyclage vers l'oxydation en phase gazeuse, et
l'eau acide est, conjointement à au moins un courant partiel du premier solvant, amenée à un dispositif de mélange et ensuite au dispositif de séparation, pour isoler du premier solvant des substances à point d'ébullition moyen, en particulier de l'anhydride maléique.

5. Utilisation suivant la revendication 4, **caractérisée en ce que** le premier solvant contient au moins un diphényle substitué ou non substitué, un éther diphénylique substitué ou non substitué ou du phtalate de diméthyle.

6. Procédé de séparation de mélanges de liquides ayant des densités différentes, dans lequel le mélange est conduit par une entrée (12) dans un récipient (11) et séjourne dedans pendant un temps nécessaire pour la séparation des phases du mélange, et dans lequel ensuite la phase lourde du mélange séparé est prélevée du récipient par une sortie (14) et la phase légère du mélange séparé par une sortie (13), l'entrée (12) étant agencée et réalisée dans le récipient (11) de telle façon que le mélange introduit frappe sur une paroi interne (16) du récipient (11) située à l'opposé des sorties (13 et 14), sous un angle α de 0° à 45° par rapport à un axe (17) qui est perpendiculaire à la paroi interne (16), **caractérisé en ce qu'**une plaque perforée (15) est agencée dans le récipient (11) entre l'entrée (12) et les sorties (13 et 14) et **en ce que** la plaque perforée (15) partage le récipient (11) perpendiculairement à l'axe (17), dans la totalité de sa section transversale.

7. Procédé suivant la revendication 6, **caractérisé en ce que** le mélange de liquides est constitué au moins d'une phase organique et d'une phase aqueuse.
